(19) Europäisches Patentamt
European Patent Office
Office européen des brevets

(11) **EP 2 914 569 B1**

(12) **FASCICULE DE BREVET EUROPEEN**

(45) Date de publication et mention
de la délivrance du brevet:
**30.08.2017 Bulletin 2017/35**

(21) Numéro de dépôt: **13801617.5**

(22) Date de dépôt: **29.10.2013**

(51) Int Cl.:
***C07C 45/52*** *(2006.01)*

(86) Numéro de dépôt international:
**PCT/FR2013/052582**

(87) Numéro de publication internationale:
**WO 2014/068241 (08.05.2014 Gazette 2014/19)**

(54) **PROCÉDÉ DE PRÉPARATION DE L'ACROLÉINE À PARTIR DE GLYCÉROL**

VERFAHREN ZUR HERSTELLUNG VON ACROLEIN AUS GLYCEROL

METHOD FOR PREPARING ACROLEIN FROM GLYCEROL

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **30.10.2012 FR 1260358**

(43) Date de publication de la demande:
**09.09.2015 Bulletin 2015/37**

(73) Titulaires:
• **Adisseo France S.A.S.**
**92160 Antony (FR)**
• **Centre National de la Recherche Scientifique**
**75794 Paris Cedex 16 (FR)**
• **Université Claude Bernard**
**69100 Villeurbanne (FR)**

(72) Inventeurs:
• **ZNAIGUIA, Raja**
**F-69100 Villeurbanne (FR)**
• **MILLET, Jean-Marc**
**F-69006 Lyon (FR)**
• **LORIDANT, Stéphane**
**F-69330 Meyzieu (FR)**
• **REY, Patrick**
**F-69003 Lyon (FR)**

(74) Mandataire: **Agasse, Stéphane et al
Cabinet GERMAIN & MAUREAU
B.P. 6153
69466 Lyon Cedex 06 (FR)**

(56) Documents cités:
**WO-A1-2011/157959     WO-A2-2010/076510
US-A1- 2008 214 384**

EP 2 914 569 B1

**Description**

La présente invention concerne un procédé catalytique de fabrication d'acroléine par déshydratation du glycérol et les applications d'un tel procédé.

On entend par glycérol, ou glycérine, un glycérol purifié ou non, d'origine naturelle, issu de l'hydrolyse d'huiles végétales et/ou de graisses animales, ou un glycérol d'origine synthétique, issu du pétrole, plus ou moins purifié ou raffiné, ou bien brut. Un glycérol purifié possède une pureté supérieure ou égale à 98%, obtenu par distillation. Un glycérol non purifié ou seulement partiellement purifié pourra être en solution dans du méthanol et/ou de l'eau lorsqu'il provient par exemple d'une transestérification de triglycérides. Dans la suite de la description, on se réfère principalement à la conversion d'un glycérol issu de la biomasse, c'est une variante préférée, mais l'invention n'y est bien entendu pas limitée et son intérêt s'étend à tous glycérols, quels que soient leurs origines et leurs degrés de pureté.

On connait des procédés de conversion du glycérol en acroléine, par déshydratation catalytique, selon la réaction :

$$HO\text{-}CH_2\text{-}CH(OH)\text{-}CH_2\text{-}OH \rightarrow CH_2\text{=}CH\text{-}CHO + 2H_2O$$

Le glycérol (appelé aussi glycérine) est depuis longtemps connu comme une source d'acroléine par transformation thermique, c'est un produit que l'on trouve largement dans la nature, sous forme d'esters (triglycérides), en particulier dans toutes les huiles et graisses animales ou végétales, ce qui en fait un réactif de départ disponible en quantité et en cela, utilisable industriellement. Il est effectivement connu que le glycérol se décompose pour donner de l'acroléine lorsqu'il est porté à des températures supérieures à 280°C. Cette réaction faiblement sélective s'accompagne de la formation de nombreux sous-produits dont l'acétaldéhyde, l'hydroxyacétone, en plus des produits d'oxydation totale CO, $CO_2$. Il est donc indispensable de contrôler la réaction de transformation du glycérol en acroléine pour éviter un gaspillage inutile de cette ressource et s'affranchir d'une séparation postérieure énergétiquement coûteuse avec un procédé de purification de l'acroléine complexe. Par ailleurs, ces impuretés, principalement des dérivés aromatiques, sont souvent à l'origine de formation de coke à la surface du catalyseur qui empoisonne ce dernier au cours du temps; il est souvent nécessaire de régénérer le catalyseur de façon à retrouver une activité catalytique satisfaisante.

Selon WO2010/076510A2, on réalise la déshydratation du glycérol en acroléine, en présence d'un catalyseur pouvant consister en un oxyde de silicium et un oxyde mixte de zirconium et de tungstène ou en un oxyde de titane et un oxyde mixte de zirconium et de tungstène; selon WO2011/157959A1, on connait un catalyseur consistant en un oxyde de zirconium, un oxyde de silicium et un oxyde de tungstène, pour effectuer cette conversion. Ces conditions permettent d'augmenter le taux de conversion du glycérol, la sélectivité en acroléine et la stabilité dans le temps de ces propriétés. US2008/214384A1 décrit un procédé de régénération d'un catalyseur de conversion du glycérol en acroléine, ce catalyseur étant acide et à base de tungstène après avoir servi dans la réaction de déshydratation du glycérol en acroléine où il a perdu de l'activité et/ou de la sélectivité. Cette régénération est réalisée par exposition dudit catalyseur seul à une atmosphère oxydante ou réductrice.

Ces performances ne sont toutefois pas rapidement obtenues, parfois seulement après 20 heures de réaction, et la nécessité d'un catalyseur encore plus efficace se fait ressentir, surtout face à une pression toujours accrue des politiques d'exploitation rationnelle des matières renouvelables.

L'invention vise à lever les problèmes rencontrés avec les catalyseurs connus dans la réaction de déshydratation du glycérol en acroléine.

L'objet de la présente invention réside dans la mise en oeuvre de catalyseurs qui tout en apportant une amélioration aux limitations ci-dessus, demeurent robustes et régénérables, permettant de produire de l'acroléine directement à partir de glycérol, notamment issu de la biomasse.

Cette alternative permet ainsi de disposer d'un procédé compétitif de synthèse d'acroléine non dépendant de la ressource pétrolière propylène à partir d'une autre matière première renouvelable.

Cette possibilité est particulièrement avantageuse pour synthétiser la méthionine ou ses analogues, comme son hydroxy-analogue (HMTBA), directement à partir de la biomasse.

Ainsi, l'invention se rapporte en outre à une application de cette réaction à la synthèse de l'aldéhyde-3-(méthylthio)propionique (MMP), du 2-hydroxy-4-méthylthiobutyronitrile (HMTBN), de la méthionine et ses analogues tels que l'acide 2-hydroxy-4-méthylthiobutanoïque (HMTBA) et l'acide 2-oxo-4-méthylthiobutanoïque, ainsi que les chélates métalliques (Zn, Ca, Cr, Zn, Cu...) et les esters de ces acides, comme l'ester isopropylique de HMTBA, à partir d'acroléine.

La méthionine, le HMTBA, les esters et chélates de celui-ci, et leurs analogues sont utilisés en nutrition animale et, dans leurs procédés industriels de synthèse, l'acroléine est généralement obtenue par oxydation du propylène et/ou du propane. L'oxydation du propylène en acroléine par l'air en présence d'eau est partielle, et le produit brut résultant, à base d'acroléine, contient aussi du propylène et du propane n'ayant pas réagi, de l'eau et des sous-produits de la réaction d'oxydation, notamment des acides, aldéhydes et alcools.

Ainsi, l'invention concerne un procédé de préparation de l'acroléine à partir de glycérol, dans lequel on réalise une déshydratation du glycérol en présence d'un catalyseur MWOA, où MWO représente un mélange d'oxydes simples et/ou d'oxydes mixtes de tungstène et d'au moins un métal M choisi parmi le zirconium, le silicium, le titane, l'alumine et l'yttrium et A représente une ou plusieurs bases de Lewis, une dite base de Lewis répondant à la formule B(R1)p(R2)q(R3)r, où B est un élément choisi parmi C, S, P, O, N et les halogénures, R1, R2 et R3 représentent, les uns indépendamment des autres, H, un groupe alkyle en C1-C6, O, OH ou OR où R représente un groupe alkyle en C1-C6, et la somme de p, q et r varie de 0 à 4. Dans une variante de l'invention, M représente au moins deux métaux choisis parmi le zirconium, le silicium, le titane, l'alumine et l'yttrium. M peut aussi représenter au moins trois ou quatre de ces métaux, voire représenter ces cinq métaux.

Comme cela ressortira des exemples, l'apport de cette ou de ces bases de Lewis aux mélanges d'oxydes précités permet de réduire de manière significative la durée de la mise en régime, tout en maintenant un taux de conversion en glycérol, une sélectivité et un rendement en acroléine élevés sur de longues périodes.

A représentant une ou plusieurs bases de Lewis, la valeur des indices p, q et r, dont la somme varie de 0 à 4, et peut ainsi être égale à 0, 1, 2, 3 ou 4, sera choisie en fonction de la nature de l'élément B.

Des bases de Lewis appropriées sont choisies parmi les anions phosphates, carbonates, carboxylates, sulfates, borates, alcoolates, les alcools et les halogénures, ainsi que leurs mélanges. Les bases préférées sont celles choisies parmi les ions phosphates $H_xPO_4^{(x-3)}$, x variant de 0 à 2, $H_3PO_4$, les ions borates, et les halogénures F⁻, Cl⁻, Br⁻, I⁻, ainsi que leurs mélanges.

Avantageusement, le rapport molaire de surface A/M varie de 0,005 à 0,5, mieux encore il varie de 0,015 à 0,09 ; A correspondant à la quantité totale de bases de Lewis.

La base de Lewis dope la surface des oxydes du catalyseur. Elle peut être ajoutée à tout moment dans la synthèse du catalyseur, et notamment au cours de la synthèse ou une fois le catalyseur formé. Si plusieurs bases de Lewis sont ajoutées, elles peuvent être incorporées, respectivement, à des étapes différentes ou non de la synthèse du catalyseur. En complément et pour préserver les performances catalytiques des catalyseurs ainsi préparés, ces mêmes agents dopants pourront éventuellement être additionnés via l'alimentation des réactifs lors du fonctionnement du réacteur.

Concernant les oxydes présents dans le catalyseur, ils sont déterminés par un rapport molaire W/somme des éléments M, différents de W ; ce rapport varie préférentiellement de 0,005 à 0,4, et mieux encore de 0,01 à 0,1.

La réaction selon l'invention peut être mise en oeuvre en phase gazeuse ou en phase liquide, de préférence en phase gazeuse. Lorsque la réaction est menée en phase gazeuse, différentes technologies de procédé peuvent être utilisées pour alimenter les réactifs, à savoir procédé en lit fixe, procédé en lit fluidisé ou procédé en lit fluidisé circulant. L'alimentation en différents réactifs, appliqués aux réacteurs susmentionnés, peut se faire de façon individuelle ou déjà sous la forme de pré mélanges. On opère à une pression de l'ordre de la pression atmosphérique et, de préférence, à une pression sensiblement supérieure. Dans les deux premiers procédés, en lit fixe ou en lit fluidisé, la régénération du catalyseur peut être séparée de la réaction catalytique. Elle peut par exemple se faire *ex situ* par les méthodes de régénération conventionnelles, comme la combustion sous air ou avec un mélange gazeux contenant de l'oxygène moléculaire. Selon le procédé de l'invention, la régénération peut se faire *in situ* car les températures et pressions auxquelles se fait la régénération sont voisines des conditions réactionnelles du procédé.

S'agissant du procédé en phase liquide, la réaction peut être réalisée dans un réacteur classique pour réaction en phase liquide sur un catalyseur solide, mais aussi dans un réacteur de type distillation catalytique en égard à la différence significative des points d'ébullition du glycérol (290°C) et de l'acroléine (53°C). On peut également raisonnablement envisager un procédé en phase liquide à une température relativement basse qui permet une distillation continue de l'acroléine produite, limitant ainsi les réactions consécutives de dégradation de l'acroléine.

Les conditions expérimentales de la réaction en phase gazeuse sont de préférence une température comprise entre 250 et 400°C à une pression comprise entre 1 et 10 bars. En phase liquide, la réaction est opérée entre 150 et 350°C et à une pression pouvant aller de 3 à 70 bars.

Un autre avantage du procédé de l'invention réside dans la forme du glycérol de départ qui peut être sous forme pure ou partiellement purifiée ou en solution, notamment aqueuse. Avantageusement, on utilise une solution aqueuse de glycérol. En solution aqueuse, la concentration du glycérol est de préférence d'au moins 1%, au mieux elle varie de 10 à 50% en poids et de préférence entre 15 et 30% en poids dans le réacteur. La concentration en glycérol ne doit pas être trop élevée dans le but d'éviter les réactions parasites qui grèvent le rendement en acroléine, comme la formation des éthers de glycérol ou des réactions d'acétalisation entre l'acroléine produite et le glycérol non converti. Par ailleurs, la solution de glycérol ne doit pas être trop diluée, en raison d'un coût énergétique rédhibitoire induit par l'évaporation du glycérol. Dans tous les cas, il est aisé d'ajuster la concentration de la solution de glycérol en recyclant partiellement ou totalement l'eau produite par la réaction considérée. L'optimisation énergétique aux bornes de la synthèse tend à récupérer la chaleur en sortie de réaction pour vaporiser le flux de glycérol alimenté au réacteur.

Un autre objet de l'invention est un procédé de fabrication de l'aldéhyde-3(méthylthio)propionique (MMP), du 2-hydroxy-4-méthylthiobutyronitrile (HMTBN), de la méthionine, de l'acide 2-hydroxy-4-méthylthiobutanoïque (HMTBA), ses chélates métalliques (Zn, Ca, Cr, Zn, Cu...), et ses esters, notamment l'ester isopropylique, et l'acide 2-oxo-4-méthylthiobutanoïque (KMB), ses chélates métalliques (Zn, Ca, Cr, Zn, Cu...), et ses esters, à partir d'acroléine, qui comprend l'étape de déshydratation du glycérol en acroléine selon l'invention. Comparativement au procédé conventionnel de fabrication de l'acroléine par l'oxydation ménagée du propylène, l'acroléine produite selon le procédé susmentionné peut contenir des impuretés différentes du procédé traditionnel, tant sous l'angle de leur quantité que de leur nature. Selon l'utilisation envisagée, synthèse de l'acide acrylique ou de la méthionine ou de son hydroxyanalogue, il pourra être envisagé de purifier l'acroléine selon les techniques connues de l'homme de l'art.

Ainsi, une fois l'acroléine directement obtenue selon l'invention ou après purification, elle est mise en réaction avec du méthylmercaptan (MSH) pour produire l'aldéhyde-3-(méthylthio)propionique (ou MMP). Dans une étape suivante, le MMP est mis en contact avec de l'acide cyanhydrique pour produire le 2-hydroxy-4-(méthylthio)butyronitrile (HMTBN). Après synthèse du HMTBN, diverses étapes réactionnelles conduisent à la méthionine, son hydroxyanalogue (HMTBA), ses chélates métalliques (Zn, Ca, Cr, Zn, Cu...), et ses esters, ou son oxoanalogue (KMB), ses chélates métalliques (Zn, Ca, Cr, Zn, Cu...), et ses esters. Toutes ces étapes à compter de la synthèse de l'acroléine sont bien connues de l'homme du métier.

Un autre objet de l'invention est l'utilisation d'un catalyseur tel que défini précédemment, pour convertir du glycérol en acroléine.

Les caractéristiques et avantages de l'invention ressortiront des exemples ci-après illustrant des catalyseurs de l'invention, leur procédé d'obtention, ainsi que leurs performances dans la réaction de conversion du glycérol en acroléine, en comparaison avec des catalyseurs de l'art antérieur.

Les catalyseurs de l'art antérieur, A, C, D et F font l'objet des exemples 1, 3, 4 et 7, respectivement. Les catalyseurs de l'invention, B, E et G font l'objet des exemples 2, 5 et 8, respectivement. La comparaison de la performance entre les catalyseurs A, C et D d'une part et les catalyseurs B et D d'autre part, est illustrée à l'exemple 6 et celle entre le catalyseur F et le catalyseur G est illustrée à l'exemple 9.

Chacun des catalyseurs A-G est caractérisée par les paramètres suivants :

- Sa surface spécifique exprimée en $m^2/g$ et mesurée par la méthode BET,
- Ses teneurs en tungstène et en métal M exprimées par un rapport molaire W/somme des éléments M différents de W, et mesurées par ICP-OES (spectrométrie d'émission à plasma inductif).

Les catalyseurs B, E et G sont en outre caractérisés par le rapport atomique de surface A/M mesuré par XPS (spectroscopie de photoélectrons X) ; A correspondant à la quantité totale de base(s) de Lewis.

La réaction de déshydratation du glycérol est conduite sur les catalyseurs indiqués, à pression atmosphérique ou une pression sensiblement supérieure, dans un réacteur droit à lit fixe. Le réacteur est placé dans un four qui permet de maintenir le catalyseur à la température de réaction qui est de 300°C. Le réacteur est alimenté avec une solution aqueuse à 20% en poids de glycérol. La solution aqueuse de glycérol est vaporisée grâce à un évaporateur C.E.M (Controlled Evaporator Mixer) Bronkhorst® en présence d'un débit d'azote. La proportion relative molaire glycérol/eau/azote est de 2,3/ 46,3/ 51,4. Le GHSV (vitesse spatiale horaire) est défini comme suit :

$$\text{GHSV = (débit molaire total x Température x R)/ (Volume catalyseur x Patm)}$$

avec Patm = 101325 Pa, Température = 25°C et le débit molaire total = débit molaire du glycérol + débit molaire de l'eau + débit molaire du gaz inerte.

**Exemple 1 : Préparation et caractérisation du catalyseur A (art antérieur)**

Le catalyseur A est de type zircone tungstée dopée à la silice, c'est-à-dire un catalyseur consistant en un mélange d'oxyde de zirconium, d'oxyde de tungstène et d'oxyde de silicium.

La préparation de ce solide comporte les trois étapes suivantes.

La première étape est la synthèse de l'hydroxyde de zirconium hydraté par co-précipitation d'une solution d'oxynitrate de zirconium $ZrO(NO_3)_2.xH_2O$ (Aldrich, >99%) et d'une solution d'ammoniaque à 28% à pH=8,8.

La deuxième étape consiste à stabiliser l'hydroxyde de zircone hydratée par des espèces siliciques par ajout d'une solution de tétra éthyle ortho silicate, TEOS, $Si(OC_2H_5)_4$ (Aldrich, 99,999%). L'hydroxyde de zircone hydraté est placé dans un ballon en téflon contenant une solution ammoniacale dont le pH est ajusté à 12 (rapport molaire Si/Zr=0,01). Le mélange est agité pendant 24h puis filtré et lavé à l'eau permutée.

La dernière étape est l'échange avec l'acide tungstique $H_2WO_4$ (Aldrich, 99%) dissous dans un peroxyde d'hydrogène à 35% à 50°C. La concentration de la solution en acide tungstique est de 0,1M. La solution d'acide tungstique est ensuite refroidie à température ambiante, et l'hydroxyde de zircone dopé à la silice est ajouté lentement. Le solide obtenu est filtré puis calciné sous air 750°C.

Sa surface spécifique est d'environ 55 m$^2$/g. La composition molaire W/M de ce catalyseur est 3,5/96,5.

**Exemple 2 : Préparation et caractérisation du catalyseur B (invention)**

Un catalyseur de type zircone tungstée dopée à la silice et au phosphore, c'est-à-dire un catalyseur consistant en un mélange d'oxyde de zirconium, d'oxyde de tungstène et d'oxyde de silicium et une base de Lewis à base de phosphate, est préparé selon l'invention. Il est synthétisé avec le même protocole que celui du catalyseur A mais, après calcination du solide, le phosphore est ajouté par imprégnation en excès à partir d'une solution de H$_3$PO$_3$ (Aldrich, >99%) à 0,2M (rapport molaire P/Zr=0,04). Le mélange est agité pendant 24h puis filtré, lavé à l'eau permutée et séché.

Sa surface spécifique est d'environ 55 m$^2$/g. Sa composition molaire W/M est identique à celle du catalyseur A.

Le rapport atomique A/M de surface est de 0,09 (avec A représentant la base de Lewis à base de phosphate).

**Exemple 3 Préparation et caractérisation du catalyseur C (art antérieur)**

Le catalyseur C est une zircone tungstée, soit un mélange d'oxyde de zirconium et d'oxyde de tungstène synthétisée par Daiichi Kigenso (référence fournisseur : Z-1104).

La surface spécifique de ce catalyseur est de 77 m$^2$/g et sa composition molaire W/M est de 3.3/96.7.

**Exemple 4 : Préparation et caractérisation du catalyseur D (art antérieur)**

Le catalyseur D est de type zircone tungstée dopée à la silice, c'est-à-dire qu'il consiste en un mélange d'oxyde de zirconium, d'oxyde de tungstène et d'oxyde de silicium. La préparation de ce solide comporte les mêmes étapes que celles du catalyseur A, à la différence de la teneur en tungstène.

Sa surface spécifique est de 63 m$^2$/g. Sa composition molaire W/M est 4,1/95,9.

**Exemple 5 : Préparation et caractérisation du catalyseur E (invention)**

Un catalyseur de type zircone tungstée dopée à la silice et au phosphore, c'est-à-dire un catalyseur consistant en un mélange d'oxyde de zirconium, d'oxyde de tungstène et d'oxyde de silicium et une base de Lewis à base de phosphate, est préparé selon l'invention. Il est synthétisé avec le même protocole que celui du catalyseur D mais, après la calcination le phosphore est ajouté par imprégnation en excès à partir d'une solution de H$_3$PO$_3$ (Aldrich, >99%) à 0,2M (P/Zr=0,04). Le mélange est agité pendant 24h puis filtré, lavé à l'eau permutée et séché.

Sa surface spécifique est de 63 m$^2$/g. Sa composition molaire W/M est identique à celle du catalyseur D.

Le rapport atomique A/M de surface est de 0,09 (avec A représentant la base de Lewis à base de phosphate).

**Exemple 6 : Evaluation des catalyseurs A, B, C, D et E dans la déshydratation catalytique du glycérol en acroléine :**

Le Tableau 1 donne les performances obtenues avec les catalyseurs A, B, C, D et E à différents temps de réaction. Les catalyseurs A, B, D et E ont été évalués avec un GHSV (vitesse spatiale horaire gazeuse) de 2900 h$^{-1}$ contre 1930 h$^{-1}$ pour le catalyseur C.

Tableau 1

| Catalyseur | A (comparatif) | | | B (invention) | | | C (comparatif) | | D (comparatif) | | | E (invention) | | |
|---|---|---|---|---|---|---|---|---|---|---|---|---|---|---|
| GHSV (h$^{-1}$) | 2900 | | | 2900 | | | 19 30 | | 2900 | | | 2900 | | |
| Temps de réaction (h) | 5 | 24 | 48 | 5 | 24 | 48 | 6 | 27 | 3 | 24 | 54 | 5 | 26 | 54 |
| Conversion du glycérol | 100 | 99 | 96 | 100 | 97 | 91 | 94 | 77 | 100 | 100 | 87 | 98 | 93 | 91 |
| Sélectivité en acroléine | 35 | 68 | 66 | 72 | 75 | 68 | 64 | 68 | 38 | 54 | 42 | 71 | 80 | 78 |
| Rendement en acroléine | 35 | 68 | 63 | 72 | 73 | 61 | 60 | 53 | 38 | 54 | 37 | 70 | 75 | 71 |
| Rendement en Acétaldéhyde | 2,4 | 2,3 | 1,4 | 2,7 | 1,8 | 1,1 | 3,8 | 2,3 | 4,1 | 2,4 | 1,7 | 3,9 | 2,9 | 1,8 |
| Rendement en propanal | 1,7 | 1,1 | 0,7 | 3,1 | 2,2 | 1,6 | 2,8 | 1,9 | 2,1 | 1,4 | 1,0 | 2,8 | 2,6 | 1,5 |
| Rendement en Acétone | 1,2 | 1,2 | 1,2 | 1,0 | 0,9 | 0,7 | 1,5 | 1,4 | 1,9 | 1,7 | 1,6 | 1,3 | 1,5 | 1,2 |
| Rendement en alcool allylique | 1,3 | 0,8 | 0,8 | 0,6 | 0,9 | 0,9 | 0,4 | 0,6 | 0,0 | 0,2 | 0,9 | 0,7 | 1,0 | 1,1 |
| Rendement en hydroxyacétone | 2,5 | 9,0 | 10,7 | 6,8 | 9,5 | 8,8 | 6,0 | 6,5 | 1,1 | 9,3 | 9,6 | 5,2 | 6,2 | 11,8 |
| Rendement en phénol | 1,1 | 0,1 | 0.1 | 0,9 | 0,2 | 0,0 | 0,8 | 0,2 | 1,5 | 0,2 | 0,0 | 3,2 | 0,9 | 0,2 |

Les catalyseurs A, B, D et E permettent une conversion totale (ou presque) du glycérol à 5h et sont nettement plus stables que le catalyseur C malgré l'utilisation d'un GHSV plus élevé. Les catalyseurs B et E (selon l'invention) permettent d'obtenir de meilleures sélectivités en acroléine à 24h. De plus, ils sont rapidement sélectifs (à 5h) et le restent jusqu'à 48-54h.

**Exemple 7 : Préparation et caractérisation du catalyseur F (art antérieur)**

Le catalyseur F est de type zircone tungstée dopée à la silice, c'est-à-dire un catalyseur comprenant un mélange d'oxyde de zirconium, d'oxyde de tungstène et d'oxyde de silicium. La préparation de ce solide comporte les mêmes étapes que celles du catalyseur A, à la différence de la température de calcination. En effet, le solide obtenu après ajout de tungstène, filtration et séchage est calciné sous air à 650°C au lieu de 750°C.

Sa surface spécifique est de 153 $m^2$/g. Sa composition molaire W/M est 11,8/88,2.

**Exemple 8 : Préparation et caractérisation du catalyseur G (invention)**

Un catalyseur de type zircone tungstée dopée à la silice, au phosphore et au fluor, c'est-à-dire un catalyseur comprenant un mélange d'oxyde de zirconium, d'oxyde de tungstène et d'oxyde de silicium et deux bases de Lewis l'une à base de phosphates, l'autre à base de fluor, est préparé selon l'invention. Il est préparé avec le même protocole que celui du catalyseur F. La seule différence pour ce catalyseur qu'il est traité dans un ballon en téflon contenant une solution ammoniacale dont le pH est ajusté à 11.9. Le mélange est agité pendant 24h sans ajout de TEOS, puis filtré et lavé à l'eau permutée. Le matériel employé et ce traitement sont responsables du dopage du solide par les ions phosphates et fluor.

Sa surface spécifique est de 91 $m^2$/g. Sa composition molaire W/M est 4,5/95,5.

Le rapport atomique P/M de surface est de 0,02. Le rapport atomique F/M de surface est de 0,04 ce qui donne un rapport atomique de surface A/M de 0,06, M représentant Zr et Si.

**Exemple 9: Déshydratation catalytique du glycérol en acroléine : évaluation des catalyseurs F et G**

Le tableau 2 donne les performances obtenues avec les catalyseurs F et G à différents temps de réaction.

Tableau 2 :

|  | F (comparatif) | | | G (invention) | | |
|---|---|---|---|---|---|---|
| GHSV ($h^{-1}$) | 2175 | | | 2900 | | |
| Temps de réaction (h) | 4 | 24 | 40 | 5 | 24 | 44 |
| Conversion du glycérol | 100 | 100 | 100 | 100 | 100 | 99 |
| Sélectivité en acroléine | 60 | 69 | 71 | 73 | 76 | 79 |
| Rendement en acroléine | 60,2 | 69,4 | 70,8 | 72,9 | 75,7 | 78,5 |
| Rendement en acétaldéhyde | 4,0 | 3,8 | 3,0 | 3,8 | 2,4 | 2,2 |
| Rendement en propanal | 6,7 | 2,8 | 2,2 | 3,3 | 2,2 | 2,4 |
| Rendement en acétone | 4,4 | 2,7 | 2,3 | 2,2 | 1,9 | 1,4 |
| Rendement en alcool allylique | 0,2 | 0,4 | 0,4 | 0,2 | 0,7 | 1,1 |
| Rendement en hydroxyacétone | 0,3 | 6,5 | 8,8 | 3,8 | 7,9 | 11,2 |
| Rendement en phénol | 0,3 | 1,8 | 0,1 | 0,6 | 0,3 | 0,1 |

Les catalyseurs F et G permettent d'obtenir des stabilités similaires. En revanche, le catalyseur G dopé aux ions phosphates et fluor (selon l'invention) permet d'obtenir de meilleures sélectivités (jusqu'à 79%) même après seulement 4h de test.

**Revendications**

1. Procédé de préparation de l'acroléine à partir de glycérol, **caractérisé en ce qu'**on réalise une déshydratation du glycérol en présence d'un catalyseur MWOA, où MWO représente un mélange d'oxydes simples et/ou d'oxydes mixtes de tungstène et d'au moins un métal M choisi parmi le zirconium, le silicium, le titane, l'aluminium, et d'yttrium et A représente une ou plusieurs bases de Lewis, une dite base de Lewis répondant à la formule B(R1)p(R2)q(R3)r, où B est un élément choisi parmi C, S, P, O, N et les halogénures, R1, R2 et R3 représentent, les uns indépendamment des autres, H, un groupe alkyle en C1-C6, O, OH ou OR où R représente un groupe alkyle en C1-C6, et la somme de p, q et r varie de 0 à 4.

**2.** Procédé selon la revendication 1, **caractérisé en ce que** M représente au moins deux ou trois métaux choisis parmi le zirconium, le silicium, le titane, l'aluminium et l'yttrium.

**3.** Procédé selon la revendication 1, **caractérisé en ce que** A est choisie parmi les ions phosphates $H_xPO_4^{(x-3)}$, x variant de 0 à 2, $H_3PO_4$, les ions borates, F⁻, Cl⁻, Br⁻, I⁻, $NH_3$, CN⁻, les ions sulfate, les ions carbonate, les ions carboxylate, les alcools, les ions alcoolate, ainsi que leurs mélanges.

**4.** Procédé selon l'une quelconque des revendications 1 à 3, **caractérisé en ce que** le rapport molaire de surface A/M varie de 0,005 à 0,5.

**5.** Procédé selon la revendication 4, **caractérisé en ce que** ledit rapport molaire de surface varie de 0,015 à 0,09.

**6.** Procédé selon l'une quelconque des revendications 1 à 5, **caractérisé en ce que** le rapport molaire W/somme des éléments M, différents de W varie de 0,005 à 0,4.

**7.** Procédé selon la revendication 6, **caractérisé en ce que** ledit rapport molaire varie de 0,01 à 0,1.

**8.** Procédé selon l'une quelconque des revendications 1 à 7, **caractérisé en ce que** le glycérol est en solution aqueuse, en une concentration d'au moins 1% en poids.

**9.** Procédé selon la revendication 8, **caractérisé en ce que** la concentration de la solution aqueuse en glycérol varie de 10 à 50% en poids.

**10.** Procédé selon l'une quelconque des revendications 1 à 8, **caractérisé en ce que** le catalyseur est régénéré.

**11.** Procédé de fabrication de l'aldéhyde-3-(méthylthio)propionique MMP, du 2-hydroxy-4-méthylthiobutyronitrile HMTBN, de la méthionine, de l'acide 2-hydroxy-4-méthylthiobutanoïque HMTBA, des esters et des chélates métalliques de ce dernier, ou du 2-oxo-4-méthylthiobutanoïque KMB, des esters et des chélates métalliques de ce dernier, à partir d'acroléine, **caractérisé en ce qu'**il met en oeuvre un procédé selon l'une quelconque des revendications 1 à 10.

**12.** Procédé selon l'une des revendications 1 à 11, **caractérisé en ce que** la réaction de déshydratation est réalisée en phase gazeuse.

**13.** Procédé selon la revendication 12, **caractérisé en ce que** la réaction de déshydratation est réalisée dans un réacteur à lit fixe, à lit fluidisé ou à lit fluidisé circulant.

**14.** Procédé selon l'une des revendications 1 à 10, **caractérisé en ce que** la réaction de déshydratation est réalisée en phase liquide.

**15.** Utilisation d'un catalyseur tel que défini dans l'une quelconque des revendications 1 à 7 et éventuellement 10, pour convertir du glycérol en acroléine.

**Patentansprüche**

**1.** Verfahren zur Herstellung von Acrolein aus Glycerin, **dadurch gekennzeichnet, dass** eine Dehydratisierung des Glycerins in Gegenwart eines MWOA-Katalysators durchgeführt wird, wobei MWO für eine Mischung aus einfachen Oxiden und/oder Mischoxiden von Wolfram und wenigstens einem Metall M steht, das aus Zirkonium, Silizium, Titan, Aluminium und Yttrium ausgewählt ist, und A für eine oder mehrere Lewis-Basen steht, wobei eine der Lewis-Basen der Formel B(R1)p(R2)q(R3)r entspricht, wobei der B ein Element ist, das aus C, S, P, O, N und den Halogeniden ausgewählt ist, R1, R2 und R3 unabhängig voneinander für H, eine Alkylgruppe mit C1-C6, O, OH oder OR stehen, wobei R für eine Alkylgruppe mit C1-C6 steht, und die Summe aus p, q und r von 0 bis 4 schwankt.

2. Verfahren nach Anspruch 1, **dadurch gekennzeichnet, dass** M für wenigstens zwei oder drei Metalle steht, die aus Zirkonium, Silizium, Titan, Aluminium und Yttrium ausgewählt sind.

3. Verfahren nach Anspruch 1, **dadurch gekennzeichnet, dass** A aus $H_xPO_4^{(x-3)}$ Phosphationen, wobei x von 0 bis 2 schwankt, $H_3PO_4$, Borationen, $F^-$, $Cl^-$, $Br^-$, $I^-$, $NH_3$, $CN^-$, Sulfationen, Carbonationen, Carboxylationen, Alkoholen, Alkoholationen sowie deren Mischungen ausgewählt ist.

4. Verfahren nach einem der Ansprüche 1 bis 3, **dadurch gekennzeichnet, dass** das Oberflächen-Molverhältnis A/M von 0,005 bis 0,5 schwankt.

5. Verfahren nach Anspruch 4, **dadurch gekennzeichnet, dass** das Oberflächen-Molverhältnis von 0,015 bis 0,09 schwankt.

6. Verfahren nach einem der Ansprüche 1 bis 5, **dadurch gekennzeichnet, dass** das Molverhältnis W/Summe der Elemente M, die von W verschieden sind, von 0,005 bis 0,4 schwankt.

7. Verfahren nach Anspruch 6, **dadurch gekennzeichnet, dass** das Molverhältnis von 0,01 bis 0,1 schwankt.

8. Verfahren nach einem der Ansprüche 1 bis 7, **dadurch gekennzeichnet, dass** das Glycerin in wässriger Lösung in einer Konzentration von wenigstens 1 Gewichts-% ist.

9. Verfahren nach Anspruch 8, **dadurch gekennzeichnet, dass** die Konzentration der wässrigen Lösung an Glycerin von 10 bis 50 Gewichts-% schwankt.

10. Verfahren nach einem der Ansprüche 1 bis 8, **dadurch gekennzeichnet, dass** der Katalysator regeneriert wird.

11. Verfahren zur Produktion von Aldehyd-3-(methylthio)propionsäure MMP, 2-Hydroxy-4-methylthiobutyronitril HMTBN, Methionin, 2-Hydroxy-4-Methylthiobutansäure HMTBA, Estern und Metallchelaten dieser letzteren, oder 2-Oxo-4-Methylthiobutansäure KMB, Estern und Metallchelaten dieser letzteren, aus Acrolein, **dadurch gekennzeichnet, dass** es ein Verfahren nach einem der Ansprüche 1 bis 10 einsetzt.

12. Verfahren nach einem der Ansprüche 1 bis 11, **dadurch gekennzeichnet, dass** die Dehydratisierungsreaktion in gasförmiger Phase durchgeführt wird.

13. Verfahren nach Anspruch 12, **dadurch gekennzeichnet, dass** die Dehydratisierungsreaktion in einem Festbett-, Wirbelschicht- oder zirkulierenden Wirbelschichtreaktor durchgeführt wird.

14. Verfahren nach einem der Ansprüche 1 bis 10, **dadurch gekennzeichnet, dass** die Dehydratisierungsreaktion in flüssiger Phase durchgeführt wird.

15. Verwendung eines Katalysators wie in einem der Ansprüche 1 bis 7 und gegebenenfalls 10 definiert, zum Umwandeln von Glycerin in Acrolein.

**Claims**

1. A method for preparing acrolein from glycerol, **characterized in that** a dehydration of the glycerol is carried out in the presence of a catalyst MWOA, wherein MWO represents a mixture of simple oxides and/or mixed oxides of tungsten and of at least one metal M selected from zirconium, silicon, titanium, alumina, and yttrium and A represents one or a plurality of Lewis bases, one said Lewis base having the formula B(R1)p(R2)q(R3)r, wherein B is a member selected from C, S, P, O, N and halides, R1, R2 and R3 represent, independently of one another, H, a C1-C6 alkyl group, O, OH or OR wherein R represents a C1-C6 alkyl group, and the sum of p, q and r ranges from 0 to 4.

2. The method according to claim 1, **characterized in that** M represents at least two or three metals chosen from zirconium, silicon, titanium, aluminum and yttrium.

3. The method according to claim 1, **characterized in that** A is chosen from phosphate ions $H_xPO_4^{(X-3)}$, x ranging from 0 to 2, $H_3PO_4$, borate ions, $F^-$, $Cl^-$, $Br^-$, $I^-$, $NH_3$, $CN^-$, sulfate ions, carbonate ions, carboxylate ions, alcohols, alcoholate ions and mixtures thereof.

4. The method according to any one of claims 1 to 3, **characterized in that** the surface molar ratio A/M ranges from 0.005 to 0.5.

5. The method according to claim 4, **characterized in that** said surface molar ratio ranges from 0.015 to 0.09.

6. The method according to any one of claims 1 to 5, **characterized in that** the molar ratio W/sum of the elements M, different from W, ranges from 0.005 to 0.4.

7. The method according to claim 6, **characterized in that** said molar ratio ranges from 0.01 to 0.1.

8. The method according to any one of claims 1 to 7, **characterized in that** the glycerol is in aqueous solution, in a concentration of at least 1% by weight.

9. The method according to claim 8, **characterized in that** the concentration of the aqueous solution in glycerol ranges from 10 to 50% by weight.

10. The method according to any one of claims 1 to 8, **characterized in that** the catalyst is regenerated.

11. The method for manufacturing 3-(methylthio) propionaldehyde MMP, 2-hydroxy-4-methylthiobutyronitrile HMTBN, methionine, 2-hydroxy-4-methylthiobutanoic acid HMTBA, esters and metal chelates thereof, or 2-oxo-4-methylthiobutanoic acid KMB, esters and metal chelates thereof, from acrolein, **characterized in that** it implements a method according to any one of claims 1 to 10.

12. The method according to any of claims 1 to 11, **characterized in that** the dehydration reaction is carried out in the gas phase.

13. The method according to claim 12, **characterized in that** the dehydration reaction is carried out in a fixed bed, fluidized bed or circulating fluidized bed reactor.

14. The method according to any of claims 1 to 10, characterized the dehydration reaction is carried out in the liquid phase.

15. A use of a catalyst as defined in any one of claims 1 to 7 and optionally 10, to convert glycerol into acrolein.

**EP 2 914 569 B1**

**RÉFÉRENCES CITÉES DANS LA DESCRIPTION**

**Documents brevets cités dans la description**

- WO 2010076510 A2
- WO 2011157959 A1
- US 2008214384 A1